# EUROPEAN PATENT APPLICATION

(11) **EP 3 815 647 A1**
(43) Date of publication of application: **05.05.2021**
(21) Application number: 19206557.1
(22) Date of filing: 31.10.2019
(51) Int. Cl.: A61F 2/04

(54) **ESOPHAGEAL PROSTHESIS**

(71) Applicant: Voigt, Kerstin-Evelyne, 4000 Liège (BE)
(72) Inventor: Voigt, Kerstin-Evelyne, 4000 Liège (BE)
(74) Representative: Quintelier, Claude

(57) **Abstract**

An esophageal prosthesis (1) comprising an inner (2), an intermediate (3) and a cover layer (4), the intermediate layer being in mechanical contact with the inner layer, the inner layer being made of a food tight inert material, which esophageal prosthesis is provided with an electro-active detection member (11,12) provided for detecting a position at the inner layer which has been contacted by food having entered the esophageal prosthesis and for generating a position signal indicating the detected position, the detection member being provided for transmitting the position signal to a processing device (5) provided for generating a stimulation signal upon receipt of the position signal, the esophageal prosthesis further comprising a contraction generating element provided for applying under control of the stimulation signal a contraction at a region of the inner layer located around the detected location in order to contract the inner layer at that region.

## Description

The present invention relates to an esophageal prosthesis comprising an inner, an intermediate and a cover layer, the intermediate layer being located between the inner and the cover layer and in mechanical contact with the inner layer, the inner layer being made of a food tight inert material.

The esophageal is the first and most important organ of the digestive system for nutrition for a human or animal being. Worldwide, there is an increasing need for esophageal surgery, as esophageal cancer and its precancerous lesions are epidemiologically becoming more important. Furthermore, there are new diagnostic devices on the market for early cancer detection with consecutive wider indications for esophageal surgery and higher case numbers. The only curative therapeutic option is, yet, oncologic surgical resection of the complete organ with its adjacent lymph nodes. Today, there are for example reported around 7000 malignant cases solely in Germany, where esophagectomy and gastric pull-up esophageal (alternatively: colonic interposition) has to be performed. In addition to the most cases of cancer, also the drinking of acids can be a reason for esophagectomy. The incidence of esophageal cancer, in particular Barrett's adenocarcinoma of the distal esophageal (esophageal adenocarcinoma = EAC), has steadily been rising in the past decades, especially in high-income countries and for younger men. A combination of molecular-genetic risk factors with a predisposition for Barrett's cancer and lifestyle parameters is supposed to be the reason for getting esophageal cancer. In spite of new oncologic developments, such as more efficient chemotherapeutic agents, antibodies against tumor cells, and, most recently, the application of immune checkpoint inhibitors within clinical trials, prognosis is poor. Today's most applied curative treatment option of esophageal cancer is surgical resection and reconstruction by a conduit, namely the gastric tube as esophageal substitute or a part of the colon, pulled of either to the upper chest or the neck, depending on the primary tumor site. Using a biological reconstruction has been rather uncommon so far, since the hallmark of esophageal function, transport of nutrition by self-peristalsis, has not proved perfectly functioning and efficient. One recent biological reconstruction has, for example, been currently described in the article of Yosake Takeoka et al. "Regeneration of esophageal using scaffold free biomineta structure created with bio-three-dimensional printer" published in Plos One of March 8, 2019.

The most frequently used esophageal substitute is the human stomach, formed to a gastric tube, with a proposed diameter of 2,5-3 cm in about 98% of cases. The less frequently applied solutions are colonic interposition and jejunal free graft. The esophageal surgery consists of three major steps:
1) Abdominal gastrolysis, in particular mobilization of the stomach and partial devascularization, with concomitant abdominal lymph node dissection:
2) Gastric tube formation by resection of the lesser curvature of the stomach with adherent lymph nodes:
3) Gastric-tube pull-up with transposition into the thorax and intrathoracic anastomosis with the remnant proximal esophageal (esophago-gastrostomy, hand-sewn or stapled).

This procedure can be performed as open, minimal-invasive or robotic esophagectomy. However, this two-hole procedure, abdominal and thoracic, is associated with high postoperative morbidity and mortality, even for younger patients otherwise fit and healthy. One of the most important reasons for postoperative complications or failure of the conduit is based on the nature of vulnerability to perfusion changes. Sufficient perfusion up to the tip of the gastric tube is provided by a small abdominal vessel only (gastroepiploic artery), sensitive to multiple influences. Even slight disturbances of perfusion might lead to major complications, such as necrosis of the esophageal substitute or anastomotic insufficiency at the site of reconnection of the remnant proximal esophageal and the gastric tube.

Both, smaller leaks of the anastomosis and total failure of the gastric tube, are potential life-threatening situations, leading to mediastinitis, systemic inflammation and severe sepsis, even in spite modern rescue and intensive care treatment. Innovative methods of intraoperative imaging of gastric tube perfusion and oxygenation assisting the search for the ideal position of the esophago-gastric anastomosis, such as hyperspectral imaging, have been tested. However, the latter methods are far away from guaranteeing a favorable outcome of esophagectomy and might improve future patient safety only slightly. Moreover, during surgery, the vagal nerves, which control the muscle movement of the esophageal have to be cut for oncologic reasons, which causes that the nerve function controlling the passage of the food is no longer available.

It is an object of the invention to provide an esophageal prosthesis enabling to reduce the risks of surgery while functioning on the basis of peristaltic requirements of nutrition and food intake.

To this purpose an esophageal prosthesis according to the present invention is characterized in that the esophageal prosthesis is provided with an electro-active detection member provided for detecting a position at the inner layer which has been contacted by food having entered the esophageal prosthesis and for generating a position signal indicating the detected position, the detection member being provided for transmitting the position signal to a processing device which is provided for generating a stimulation signal upon receipt of the position signal, the esophageal prothesis further comprising a contraction generating element connected to the processing device, the contraction generating element being provided for applying under control of the stimulation signal a contraction at a region of the inner layer located around the detected location in order to contract the inner layer at that region. The presence of the electro-active detection member enables to detect the position at the inner layer, which has been contacted by food having entered the esophageal prosthesis. The thus generated position signal enables to generate the stimulation signal, which on his turn activates the contraction generating element for applying a contraction at the region of the inner layer located around the detected location. This contraction then simulates the physiologic myoelectric waves of the human esophageal, thereby allowing the food to pass from the mouth to the digestive system. Providing in such a way the contraction of the inner layer offers a solution to the problem that during surgery, the vagal nerves, which control this muscle movement, have to be cut for oncologic reasons, meaning that the muscle action is now provided by the esophageal prosthesis.

Preferably the electro-active detection member is applied at the intermediate layer. In such a manner the detection member is not in direct contact with the food but close enough to the inner layer to detect the pressure applied by the presence of food in the esophageal.

A first preferred embodiment of the esophageal prosthesis according to the present invention is characterized in that the detection member comprises an electric capacity having a value, which is dependent of the position where it is applied along a length of the esophageal prosthesis. Using such an electric capacity provides a reliable and accurate way to detecting the position of the food having entered the esophageal.

A second preferred embodiment of the esophageal prosthesis according to the present invention is characterized in that the electric capacity is build up by a plurality of active foil stripes successively applied along the length of the esophageal prosthesis and galvanically separated from each other, each foil stripe being provided for being separately fed by electric power. The use of a plurality of active foil stripes galvanically separated from each other, whereas each foil stripe is separately feed by electric power enables an accurate determination of the position, where the food has entered the esophageal prosthesis as it divides, as if to say the esophageal prosthesis in separate strokes, which can be individually identified.

A third preferred embodiment of the esophageal prosthesis according to the present invention is characterized in that the intermediate layer comprises a foil, said foil, being provided to be locally contracted under control of the stimulation signal. This provides a reliable solution for enabling the contraction of the esophageal prosthesis.

A fourth preferred embodiment of the esophageal prosthesis according to the present invention is characterized in that the inert material of the inner layer is made of materials chosen among polymer, Peek, PEI, PPSU, polycarbonates, medical-grade liquid silicone rubber, titanium or ABS. Those materials are biocompatible.

The invention will now be described with reference to the drawings showing a preferred embodiment of the esophageal prosthesis according to the present invention. In the drawings:
Figure 1 shows a cross section in length direction through the esophageal prosthesis;
Figure 2 shows the application of a plurality of active foil stripes applied along a length of the intermediate layer;
Figure 3 shows an embodiment where the electric capacity varies over the length of the intermediate layer;
Figure 4 shows an embodiment of the cover layer of the esophageal prosthesis;
Figure 5 shows the movement of the food being transported through the esophageal;
Figure 6 illustrates the operation of the processing device; and
Figure 7 illustrates the contraction signal.

In the figures a same reference number has been allotted to a same or similar element.

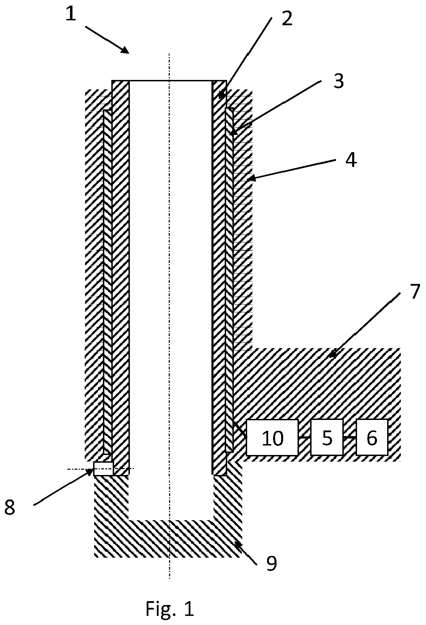

Figure 1 shows a cross section in length direction through the esophageal prosthesis 1 according to the invention. The esophageal prosthesis comprises an inner 2, an intermediate 3, and a cover 4 layer. The intermediate layer is located between the inner and the cover layer and in mechanical contact with the inner layer. The word layer covers a single layer of material as well as an embodiment where each of the layers are made of multiple of foils or sheets, the latter being applied on each other either by stacking them in a same direction or with an angle among each other.

The inner layer 2 is made of a food tight inert material and preferably smooth at the inside in order not to obstruct the food passage travelling across the channel delimited by the inner layer. The inert material is preferably made of an inert polymer, which will have the mechanical properties most similar to the human esophageal and having limited risk of rejection by the body. One suitable inert material for the inner layer is silicone. The length of the esophageal prosthesis is preferably between 35 and 45cm, depending on the morphology of the patient. The inner diameter of the esophageal prosthesis is preferably between 25 and 28mm. There are of course other potential materials for the inner layer such as for example PEEK, PEI (Ultem), and PPSU; polycarbonates; medical-grade liquid silicone rubber (LSR); and 3D-printed titanium and ABS-like WaterShed XC 11122.

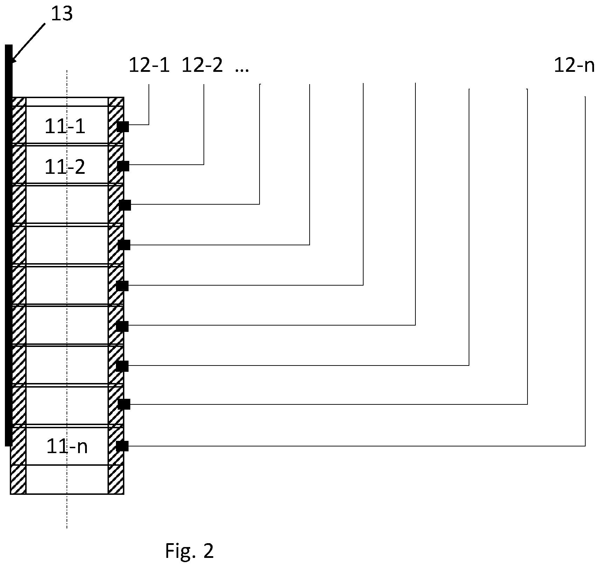

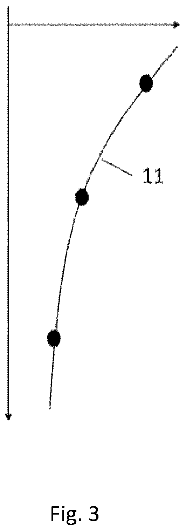

The intermediate layer 3 is the part of the esophageal prosthesis that provides the muscle action. The esophageal prosthesis is provided with an electro-active detection member, such as illustrated in the figures 2 or 3, and which is provided for detecting a position at the inner layer, which has been contacted by food having entered the esophageal prosthesis. The electro-active detection member is preferably applied on the intermediate layer. The intermediate layer further comprises a contraction generating element provided for applying a contraction on that intermediate layer.

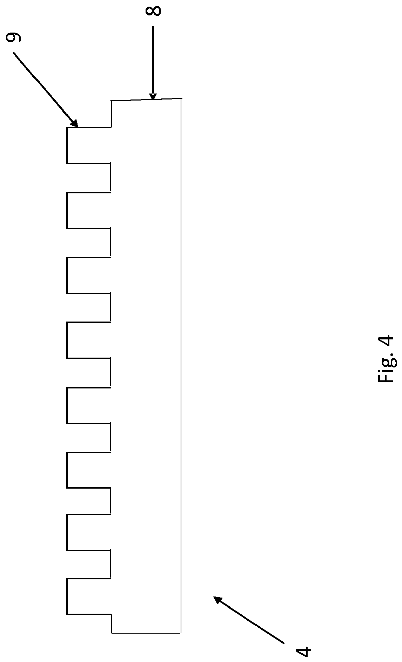

The cover layer 4 serves as an envelope wrapped around the inner and intermediate layer and preferably comprises a processing device 5 and a battery 6 as will be described hereunder. The battery is preferably lodged in a tight pocket 7 so as be isolated and does not get into touch with the body fluid of the body in which the esophageal prosthesis is implanted or with the food crossing the esophageal. The cover layer is formed out of a structured polymer as illustrated in figure 4. This structured polymer will determine the flexibility and the strength of the esophageal prosthesis. The cover layer is preferably printed using an additive manufacturing technology. A well-suited material for the cover layer is for example PEEK since it has a convenient stiffness, is inert to the human body and can be printed with 3D printing technology. The cover layer 4 is also used to embed the complete signal and ground contact lines as shown in figure 2 or 3.

The cover layer 4 defines the mechanical properties in terms of stiffness and flexibility of the esophageal prosthesis. Therefore, the outer surface is preferably structured as shown in figure 4. The outer surface comprises a basis layer 8 provided reinforcing ribs 9, which rigidifies the cover layer. The cover layer might be either glued to the intermediate layer 3 or welded axially once it is wrapped around the intermediate layer. A step-up converter 10 for providing a high voltage is preferably applied on the cover layer.

As shown in figure, 2 the esophageal prosthesis is provided with an electro-active detection member 11,12 provided for detecting a position at the inner layer which has been contacted by food having entered the esophageal prosthesis. The electro-active detection member is preferably applied on the intermediate layer 3. Alternatively, the electroactive detection member could also be integrated into the intermediate layer, or directly 3D-printed on the inner layer or on the cover layer. The detection member preferably comprises an electric capacity having a value, which is dependent of the position where it is applied along a length of the esophageal prosthesis. In the embodiment shown in figure 2, the electric capacity comprises a plurality of active foil stripes 11-1, 11-2, ...,11-n, successively applied along a length of the intermediate layer and galvanically separated from each other. Each foil stripe having a dedicated contact element 12-1, 12-2, ..., 12-n so as to be separately feed by electric power. A common ground 13 is applied against the cover layer 4 so as to create an electrical voltage over the inner layer.

In the embodiment shown in figure 3, the electric capacity 11 varies over the length of the esophageal prosthesis. So, the capacity value is the highest at the entrance of the esophageal prosthesis and decreases along the length of thereof. Analogously as for the embodiment of figure 2, a common ground 13 is applied. The change of the capacity value is for example realized by varying the dielectric value or by application of a curved capacitive layer on the intermediate layer.

Instead of varying the capacity value, it could also be envisaged to use a shunt having a resistance value which varies, for example by using a foam layer having a carbon concentration varying over the length of the esophageal prosthesis.

The esophageal prosthesis further preferably comprises a valve 18 applied within the volume delimited by the inner layer and which will serve as anti-reflux contraption in order to prevent acid juice from the stomach coming up into the esophageal prosthesis and from there up to the respiratory system with consecutive silent aspirations. The valve being preferably a spool valve held by the cover layer. The valve is normally in its closed state. As soon as food is pushed through the esophageal prosthesis there will be a pressure difference applied on the valve, which will open the valve till a valve stop is reached. When the food has passed, the valve will be closed again from the pressure on the stomach side.

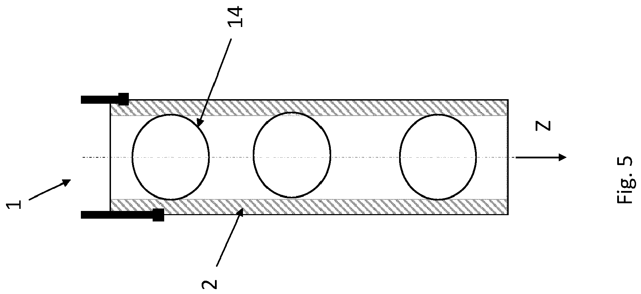

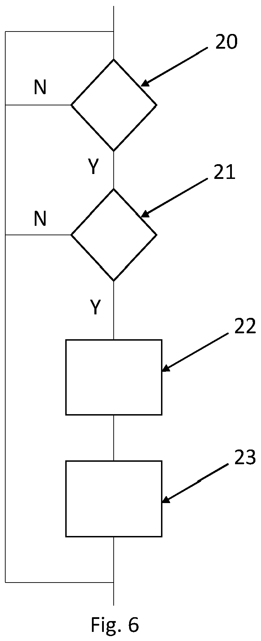

The detection member enables to detect a position at the inner layer, which has been contacted by food having entered the esophageal prosthesis. This is realized by applying an electric voltage over the capacity and the common ground. As illustrated in the figures 5 and 6, when food 14 has entered the esophageal prosthesis, a pressure will be applied due to a local expansion of the inner layer at the position, where the food contacts that inner layer. As the inner layer is in mechanical contact with the intermediate layer, the applied pressure will change the capacity at that position leading to a change in the measured value of the electric current circulating through the capacity, in a manner very similar to the piezo effect. The processing device 5, for example formed by a microprocessor with embedded software and which functioning is illustrated by the flowchart shown in figure 6, monitors the signals received from the detection member. In the embodiment illustrated in figure 2 the position is detected by recognizing on which of the foil's strips 11-i the pressure was applied. In the embodiment illustrated in figure 3, the position is determined by measuring the value of the current flowing through the capacity. Indeed, as the capacity value over the length, the value of the measured current will indicate the position at which the food applied the pressure. The capacity will only provide one signal at time, but the signal amplitude will change successively as the food travels across the esophageal prosthesis.

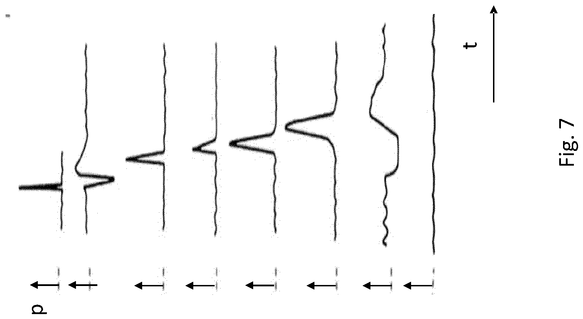

Once this position has been determined, the detection member will generate a position signal indicating the detected position. The detection member is provided for transmitting the position signal to a processing device. The processing device monitors 20 the received position signal and preferably verifies (21) if the received value can be considered as useful, for example by verifying if this value exceeds a predetermined threshold. If the value is less than the threshold value (21, N), it is not taken into consideration. Upon receipt of the position signal and after having determined that the received signal exceeds the threshold value (21, Y) the processing device will generate 22 a stimulation signal which will be supplied to a contraction generating element connected to the processing device. Based on the position signal indicating the position where the pressure was applied, the processing device will also determine 23, at which region of the esophageal prosthesis the contraction has to be applied in order to take care that the contraction is applied at the region where the food was detected. The contraction generating element is provided for applying under control of the stimulation signal a contraction at a region of the inner layer located around the detected location in order to contract the inner layer at that region. Figure 7 provides an example of the values of the stimulation signals.

This contraction is for example realized by applying a voltage to the intermediate layer. As soon as a voltage is applied to the intermediate layer, it can at the region where it is applied contract the intermediate layer and execute a muscle action. This action pushes the food across the channel formed by the inner layer and is the active part of the esophageal prosthesis. The contraction is a function of the region of the inner layer located around the detected location. The intermediate layer moves under the application of high voltage to the inside of the channel and thus reduces the cross section. This movement successively pushes the food through the channel. The voltage could be ideally identical for all foil strips or with more effort applicable for each individual stripe. If the multi stripe approach is used, each of them has to be contacted individually.

The application of the contraction and the determination of the position need energy. Therefore, the battery 6 will preferably be of the Qi type, which allows a charging without any contact by electromagnetic waves. The intermediate layer needs high voltage in the kV domain, in particular a voltage higher than 1KV. Therefore, the battery voltage must be converted to high voltage via the embedded step-up converter 10.

The prosthesis will have two anastomotic sites. The first will be orally with the remnant esophageal and the second aborally with the kardia being the entrance of the stomach, or the stomach itself. Both will preferably by end-to-end anastomoses, hand-sewn, preferably with non-resorbable sutures, or stapled with titanium staples of varying sizes. All layers of the esophageal prosthesis will be sutured and anatomized with the remnant esophageal and the proximal stomach by one-layer stitches.

The esophageal prosthesis will, thus, give patients a realistic chance to survive in cases of cancer and various other reasons of benign and malignant esophageal disease.

## Claims

1. An esophageal prosthesis (1) comprising an inner (2), an intermediate (3) and a cover (4) layer, the intermediate layer being located between the inner and the cover layer and in mechanical contact with the inner layer, the inner layer being made of a food tight inert material, **characterized in that** the esophageal prosthesis is provided with an electro-active detection member (11,12) provided for detecting a position at the inner layer which has been contacted by food having entered the esophageal prosthesis and for generating a position signal indicating the detected position, the detection member being provided for transmitting the position signal to a processing device (5) which is provided for generating a stimulation signal upon receipt of the position signal, the esophageal prothesis further comprising a contraction generating element connected to the processing device, the contraction generating element being provided for applying under control of the stimulation signal a contraction at a region of the inner layer located around the detected location in order to contract the inner layer at that region.

2. The esophageal prosthesis as claimed in claim 1, **characterized in that** the electro-active detection member is applied at the intermediate layer.

3. The esophageal prosthesis as claimed in claim 1 or 2, **characterized in that** the detection member comprises an electric capacity (11) having a value, which is dependent of the position where it is applied along a length of the esophageal prosthesis.

4. The esophageal prosthesis as claimed in claim 3, **characterized in that** the electric capacity is build up by a plurality of active foil stripes (11-1, 11-2, ... 11-n) successively applied along the length of the esophageal prosthesis and galvanically separated from each other, each foil stripe being provided for being separately fed by electric power.

5. The esophageal prosthesis as claimed in claim 3, **characterized in that** the electric capacity varies over the length of the esophageal prosthesis.

6. The esophageal prosthesis as claimed in anyone of the claims 1 to 5, **characterized in that** the intermediate layer comprises a foil, said foil being provided to be locally contracted under control of the stimulation signal.

7. The esophageal prosthesis as claimed in anyone of the claims 1 to 6, **characterized in that** the inner layer has mechanical properties similar to the one of an esophagus of a living body, in particular a human body.

8. The esophageal prosthesis as claimed in anyone of the claims 1 to 7, **characterized in that** the esophageal prosthesis is tubular shaped and has a diameter situated between 25 and 28 mm and a length situated between 35 and 45 cm.

9. The esophageal prosthesis as claimed in anyone of the claims 1 to 8, **characterized in that** said inert material of the inner layer is made of materials chosen among polymer, Peek, PEI, PPSU, polycarbonates, medical-grade liquid silicone rubber, titanium or ABS.

10. The esophageal prosthesis as claimed in anyone of the claims 1 to 9, **characterized in that** the esophageal prosthesis is made by 3D printing or by gluing of the layers upon each other.

11. The esophageal prosthesis as claimed in anyone of the claims 1 to 10, **characterized in that** cover layer is made of a structured polymer, in particular PEEK.

12. The esophageal prosthesis as claimed in anyone of the claims 1 to 11, **characterized in that** the processing device is located in the cover layer.

13. The esophageal prosthesis as claimed in anyone of the claims 1 to 12, **characterized in that** the cover layer comprises a battery storage housing (7) provided for storing an electrical battery.

14. The esophageal prosthesis as claimed in anyone of the claims 1 to 13, **characterized in that** it comprises a step-up converter (10) provided for generating a high voltage of at least 1 kV.

15. The esophageal prosthesis as claimed in anyone of the claims 1 to 14, **characterized in that** it comprises a valve (18) applied within the volume delimited by the inner layer.
